# EUROPEAN PATENT APPLICATION

(11) **EP 3 677 184 A1**
(43) Date of publication of application: **08.07.2020**
(21) Application number: 19150018.0
(22) Date of filing: 02.01.2019
(51) Int. Cl.: A61B 6/00, A61B 5/00, A61B 5/055, A61B 5/11

(54) **STRESS SUPPORT SYSTEM**

(71) Applicant: Koninklijke Philips N.V., 5656 AG Eindhoven (NL)
(72) Inventor: JOHNSON, Mark Thomas, 5656 AE Eindhoven (NL); HELLE, Michael Günter, 5656 AE Eindhoven (NL); VOGTMEIER, Gereon, 5656 AE Eindhoven (NL); SISODIA, Rajendra Singh, 5656 AE Eindhoven (NL); VUPPALA, Sunil Kumar, 5656 AE Eindhoven (NL)
(74) Representative: de Haan, Poul Erik

(57) **Abstract**

The present invention relates to diagnostic imaging. In order to provide an improved, efficient, and/or fast procedure for adapting the physical stress during diagnostic imaging, a stress support system is provided. The stress support system comprises a stress determination device and at least one stress inducing device with a support device and a control device. The support device is attachable to a patient and configured to apply a physical stress to a body part of the patient. The stress determination device is configured to determine a required stress level for a medical scan of the body part. The control device is configured to modify the support device to adjust the applied physical stress to the required stress level.

## Description

### FIELD OF THE INVENTION

The present invention relates to diagnostic imaging. In particular, the present invention relates to a stress support system, a medical imaging system as well as a method for imaging a body part of a patient.

### BACKGROUND OF THE INVENTION

Medical scans may be carried out when a body part, e.g. limbs, spine, is at rest. In order to initiate certain physiological problems it may be required that the body part is dynamically stressed, e.g. doing a cycling motion, whilst being imaged. A healthcare professional may adapt the stress e.g. on a limb of a patient between scans for taking repeated images at different physical stress situations. This may take time and may result in unwanted changes of the position of the patient.

### SUMMARY OF THE INVENTION

There may be a need to provide an improved, efficient, and/or fast procedure for adapting the physical stress during diagnostic imaging.

The object of the present invention is solved by the subject-matter of the independent claims, wherein further embodiments are incorporated in the dependent claims. It should be noted that the following described aspects of the invention apply also for the stress support system, to the medical imaging system and to a method for operating the stress support system.

A first aspect of the invention relates to a stress support system for diagnostic imaging. The stress support system comprises a stress determination device and at least one stress inducing device with a support device and a control device. The support device is attachable to a patient and configured to apply a physical stress to a body part of the patient. The stress determination device is configured to determine a required stress level for a medical scan of the body part. The control device is configured to modify the support device to adjust the applied physical stress to the required stress level.

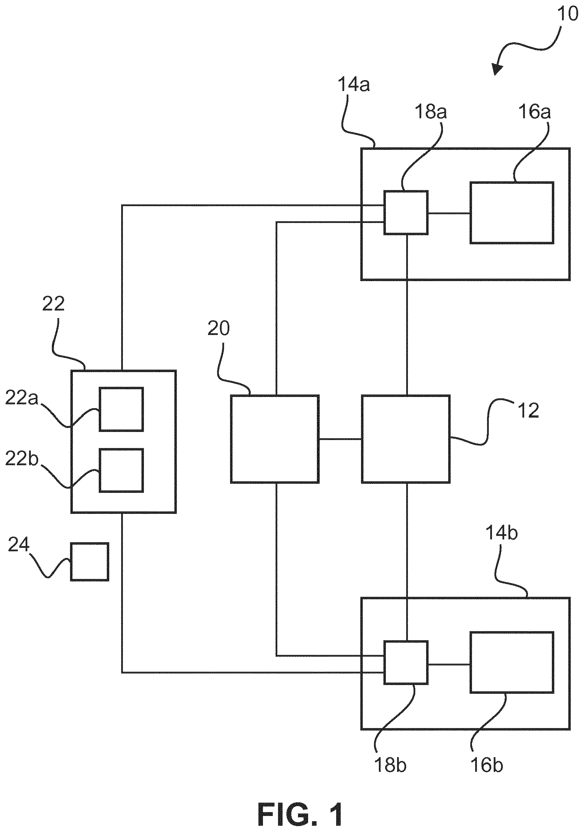

In other words, a stress support system is proposed to induce a static or dynamic physical stress to a body part, such as a limb, arm, leg, or back, of a patient for imaging purposes. As will be explained hereafter and particularly with respect to the exemplary embodiment in Fig. 1, astronaut type training devices, such as tension strips, exoskeletons or braces may be added to induce static or dynamic physical stress to a body part to image e.g. a limb whilst the limb is under stress. The patient may be connected to the support device, such as a brace, tension strip, exoskeleton, etc., such that the body part to be imaged is tensioned prior to the imaging. This may be done by a healthcare professional prior to the imaging. During the imaging, it is possible to dynamically adjust the support device in order to adjust the stress on the body part between scans with no or less operator interference as the stress levels may be predetermined, i.e. determined prior to the imaging, by the stress determination device. It is thus possible to adapt the stress on the body part and to take repeated images with the medical imaging device at different stress situations, whilst the body part is in essentially the same position or in essentially the same motion state. Accordingly, there is less operator interference between scans, thereby reducing time for positioning the patient and preventing unwanted changes of the position of the patient.

The "stress determination device" as used herein may refer to a data processing element such as a microprocessor, microcontroller, field programmable gate array (FPGA), central processing unit (CPU), digital signal processor (DSP) capable of providing a signal to the control device via a physical cable, such as a standard universal serial bus (USB), a Bluetooth based communication channel, a wireless connection or another form of data connection. The stress determination device may determine one stress level for a medical scan. The stress determination device may optionally determine a plurality of stress levels for a series of medical scans and/or a minimum-maximum range of the stress levels for the series of medical scans. As will be explained hereafter, the stress determination device may be coupled to a user interface to receive a user input indicating these parameters. In another example, the stress determination device may determine these parameters based on historical data of the patient or similar patients. It is noted that these parameters may be predetermined, i.e. determined prior to the imaging. Thus, during the imaging the stress induce device may adapt the static or dynamic stress in an autonomous manner with no or less operator interference.

The "support device" as used herein may be a support device for inducing a static stress or a support device for inducing a dynamic stress. The support device for inducing a static stress may include e.g. a brace, a tension strip, an exoskeleton, etc., which is attachable to the patient to stress the body part statically, such that the body part to be imaged is tensioned prior to and during the imaging. The support device for inducing a dynamic stress may include e.g. a brace, an exoskeleton, etc., which is attachable to the patient to stress the body part dynamically, such that the body part to be imaged is tension whilst in motion during the imaging. The support device for inducing a dynamic stress may also include a cycling type device. The load on the pedals can be dynamically increased, for example by adjusting a braking system to stress the body part e.g. a limb dynamically.

The term "attachable" as used herein may refer to connecting the patient to the support device. For example, the support device is a wearable device, such as an exoskeleton or a brace. However, the support device may also be a non-wearable device, such as a cycling type device.

The "control device" as used herein may refer to a motorized device that receives instructions to modify the support device. If the support device is a mechanical device, such as a mechanical brace, the control device may adjust the position of e.g. a spring in order to increase or decrease the static stress level. If the support device is an elasticated device, such as a tension strip, the control device may adjust an anchoring position of a support of the band by e.g. tightening or loosening a screw thread. If the support device is a cycling type device, the control device may adjust the load on the pedals to increase or decrease the stress level. The control device may be connected to a user interface to allow a user to start and stop applying the physical stress. The control device may be connected to a safety means, such as a pain sensing device. The control device may be triggered to stop applying the physical stress or to re-adjust the applied physical stress, if an excessive pain level is detected.

The medical imaging device may be a magnetic resonance imaging (MRI) scanner or a computed tomography (CT) scanner. In case of MRI, the support device may be MRI compatible. This may be achieved e.g. by using dedicated pressure sleeves that can force the limb into a predefined position when filled with air or liquid. In another example, the support device may use non-magnetic material, e.g. polymers, for a mechanical brace or exoskeleton that can be operated by an extended axis from the control device 18a, 18b, which is outside the magnetic field. In case of CT, non-metallic material e.g. carbon fiber based materials can be used for mechanical stability.

According to an embodiment of the invention, the at least one stress inducing device comprises a static stress inducing device with a first support device and a first control device. The stress determination device is configured to determine a required static stress level for a medical scan. The first support device is attachable to the patient and configured to apply a static physical stress to the body part. The first control device is configured to modify the first support device to adjust the applied static physical stress to the required static stress level. Alternatively or additionally, the at least one stress inducing device comprises a dynamic stress inducing device with a second support device and a second control device. The second support device is attachable to the patient and configured to induce a dynamic stress to the body part while being in motion. The stress determination device is configured to determine a required dynamic stress level for a medical scan. The second control device is configured to modify the second support device to adjust the dynamic physical stress to the required dynamic stress level. The medical imaging device is configured to perform a medical scan of the body part to acquire dynamic image data, when the required dynamic stress level is reached.

In other words, the first support device may be provided to induce static stress to a body part, such as a limb or the back, to image the body part whilst the body part is under stress. In order to achieve this, the patient is connected to the first support device, such as a brace, tension strip, exoskeleton, etc., such that the body part to be imaged is tensioned prior to the imaging.

The second support device may be provided to induce dynamic physical stress to a body part to image the body part whilst the body part is both in motion and under stress, for example carrying out a cycling motion. In order to achieve this, the patient may be connected to the second support device, such as a brace or exoskeleton, to stress the body part dynamically, such that the body part to be imaged is tensioned whilst in motion during the imaging.

According to an embodiment of the invention, the stress determination device is configured to determine a required direction of the physical stress applied to the body part for a medical scan. The control device is configured to modify the support device to adjust the applied physical stress to the required direction.

In addition to the stress value, the direction of the stress inducing motion could be modified to scan, e.g. the angular stress curve.

According to an embodiment of the invention, the stress determination device is configured to determine at least one of the following: a plurality of required stress levels for a series of medical scans, and a minimum-maximum range of the required stress levels to be applied to the body part. The control device is configured to modify the support device to adjust the applied physical stress to the plurality of required stress levels for the series of medical scans and/or to adjust the applied physical stress within the minimum-maximum range of the required stress levels.

Accordingly, the stress support system can adapt the static/dynamic stress on the body part for a series of medical scans with minimal operator interface between scans. This may advantageously save time and avoid unwanted changes in position of the patient.

According to an embodiment of the invention, wherein at least one of the following is determined based on historical data of the patient or similar patients: one or a plurality of the required stress levels for one or a series of medical scans, and a minimum-maximum range of the required stress levels to be applied to the body part.

In other words, historical data of the patient or similar patients may be used e.g. with machine learning algorithms to define the minimum and maximum range of the stress. The plurality required stress levels may also be determined based on the historical data. For example, machine learning algorithm may use the stress increase and decrease curves to predict the acceptable personalized stress levels for a series of medical scans.

This may advantageously allow the stress determination device to determine these parameters without any user input. This may be beneficial for an autonomous image acquisition procedure.

According to an embodiment of the invention, the stress support system comprises a user interface. The user interface is configured to receive a user input to provide at least one of the following: i) defining at least one required stress level for a medical scan, ii) defining a minimum-maximum range of the required stress levels to be applied to the body part, iii) starting applying a physical stress, and iv) stopping applying a physical stress. The stress determination device is connectable to the user interface and configured to determine one or a plurality of required stress levels and/or a minimum-maximum range of the required stress levels based on the user input. Alternatively or additionally, the control device is connectable to the user interface and configured to start or stop modifying the support device to apply the physical stress based on the user input.

The user interface may be a personal computer (PC), a handheld device, a button etc., connectable to the control device and/or stress determination device via a physical cable or wirelessly.

The user interface may be a dedicated patient interface to provide a tolerable stress level. The dedicated patient interface may also allow the patient to terminate a scan sequence if the increase of the stress causes excessive pain. Alternatively or additionally, the user interface may a user interface for a healthcare professional e.g. for defining at least one required stress level for a medical scan and defining a minimum-maximum range of the required stress levels to be applied to the body part.

According to an embodiment of the invention, the stress support system comprises at least one safety means. The at least one safety means is configured to provide at least one safety parameter for limiting a maximum applied stress level. The control device is configured to stop applying the physical stress to the body part of the patient or to re-adjust the applied physical stress level, if the at least one provided safety parameter matches a predefined criterion.

In other words, the safety means may be used to prevent the patient from an excessive pain caused by a high stress level. This may reduce the risk of injury of the patient during the imaging under increasing physical stress. In particular, this may be beneficial for situations where the patient cannot easily express their pain to a healthcare professional - either because of physical or mental limitations or that there is no healthcare professional available. The predefined criterion may be e.g. whether a predefined threshold is reached.

According to an embodiment of the invention, the at least one safety means comprises an applied stress measuring device configured to determine an absolute value of the applied stress level as the at least one safety parameter. The control device is configured to stop applying the physical stress to the body part of the patient or to re-adjust the applied physical stress level, if the absolute value of the applied stress level matches a predefined criterion.

The applied stress measuring device may sense the stress of tissue, muscle, or tendon using shear wave electrography or similar acousto-elasticity techniques using an ultrasound sensor to measure the change in stress during imaging.

According to an embodiment of the invention, the at least one safety means comprises a pain sensing device configured to measure a biometric signal indicative of an acute pain level of the patient as the at least one safety parameter. The acute pain level is determined by the measured biometric signal based on a pain-biometrics model, which defines a relationship between an acute pain and a biometric signal based on historical data of the patient or similar patients. The control device is configured to stop applying the physical stress to the body part of the patient or to re-adjust the applied physical stress level, if the determined acute pain level matches a predefined criterion.

As the acute pain is detected, the control device can take a corrective action before the patient suffers a prolonged pain. This may be beneficial for the patient who cannot easily express their pain to the healthcare professional. This may also be beneficial for the situation where no healthcare professional is available.

The "pain sensing device" as used herein may be a device, which measures a vital sign, which rapidly responds to an acute pain. The vital sign may include the galvanic skin conductance (GSC) of the patient, which is measured using two electrodes and a high-impedance amplifier attached to a part of the patient's body. The GSC may have the advantage that a rapid and large increase of skin conductance is recorded around seconds after an acute pain event. Corrective action may be taken before the patient suffers a prolonged pain. In another example, the pain sensing device may be a device which measures another biometric signal indicative of pain. The pain sensing sensor may be integrated in the static or dynamic stress inducing device. The pain sensing device may be located between the tissue of the patient and support device - for example on the inside of the brace or exoskeleton. Alternatively, the pain sensing sensor is worn separately at a preferred part of the patient's body, e.g. on the wrist, fingers or foot.

The "pain-biometrics model" as used herein may be e.g. a machine learning model, such as regression and deep learning based machine learning model. The biometric signal measured by the pain sensing device may be passed through machine learning technique to dynamically identify the pain levels.

According to an embodiment of the invention, the stress support system comprises a residual stress sensing device. The residual stress sensing device is configured to measure a resultant stress due to an external pressure for correlating the determined acute pain level.

The residual stress sensing device may be an ultrasound sensor, e.g. attached to the limbs, to measure the resultant stress, and correlate to the level of pains using acousto-elasticity scans for re-construction of scan image. In other words, ultrasound sensors are used for performing shear wave scanning, i.e. acousto-elasticity scan to measure the elasticity and stress of the tissues or tendon and to accurately measure the resultant additional stress at tissue or tendon due to external pressure for imaging.

According to an embodiment of the invention, the at least one safety means comprises an image based feedback for providing at least one of the following as the at least one safety parameter: an imaging sequence, an image quality, and an imaging parameter.

In other words, an image based feedback may be used as limiting parameters in the image domain to give maximum limits for stress values before adding additional risk to the patient. For some scans, the tissue, muscle, or limbs has to be under stress, for e.g. injury during play, which requires that unless a muscle is under stress, the diagnosis of underlying issue is difficult. The max stress is defined as the stress which can re-produce the underlying diagnostic condition or the pain bearer of the patient. Manipulation can be realized within a certain parameter space, defined by the imaging sequence, image quality, and the pain level of the patient. Via an iterative feedback loop between the imaging system and patient, increased image quality may be assured at a tolerable pain level of the patient. Imaging parameters may include e.g. minimum and maximum distances of muscles, bones, etc. This may also allow the realization of autonomous imaging or partially autonomous imaging.

A second aspect of the invention relates to a medical imaging system. The medical imaging system comprises a stress support system as described above and below and a medical imaging device. The stress support system is configured to apply a physical stress to a body part of a patient at a required stress level for a medical scan. The medical imaging device is configured to perform a medical scan of the body part to acquire image data, when the required stress level is reached.

In this way, one or a sequence of images of the body part can be measured during motion or in rest and the stress level can be modified in a defined way e.g. within a minimum-maximum range to image the reaction of the muscles, tissue, bone, and the movement direction of the extremities. In case of mechanically forced "misalignment", the body reaction and the degree of freedom in the joints linked to the muscle stress could be imaged. This may allow autonomous, or partially autonomous, image acquisition of a body part under a series of physical stress levels.

According to an embodiment of the invention, the stress determination device is configured to determine a plurality of required stress levels for a series of medical scans. The medical imaging device is configured to perform a series of medical scans of the body part at the plurality of required stress levels. The medical imaging device is configured to be gated in a manner such that the image data of the series of medical scans are reconstructed at the same position of the body part.

This may ensure that images can be reconstructed in essentially the same position or in essentially the same motion state.

According to an embodiment of the invention, the medical imaging device is configured to perform a series of medical scans in synchronization with a motion state and/or a position of the body part.

In other words, the scanning moment is synchronized to the dynamic motion using extremely reproducible positioning. The motion state may refer to a specific time during motion or at specific location, e.g., during cycling, when the knee is stress maximally.

According to an embodiment of the invention, a trigger signal is provoked when the support device is in a predefined position. The medical imaging device is configured to perform a medical scan upon receiving the trigger signal.

According to an embodiment of the invention, the medical imaging device further comprises a motion tracking device. The motion tracking device is configured to track a motion state and/or a position of the body part whilst being in motion. The medical imaging device is configured to perform a medical scan of the body part if a desired motion or a desired position is reached.

The motion tracking device may include sensors or markers that can be tracked e.g. with a camera.

According to an embodiment of the invention, the stress support system further comprises a force sensing device. The force sensing device is configured to measure a force between the body object and the attached support device. The medical imaging device is configured to perform a medical scan of the body part if a desired force is reached. The force sensing device may be an air paid, which may be arranged between the body object and the attached first or second support device that touch.

According to an embodiment of the invention, the medical imaging device is configured to provide a dedicated sequence to track a motion state and/or a position of the body part whilst being in motion. The medical imaging device is configured to perform a medical scan of the body part if a desired motion state and/or a desired position is reached.

According to an embodiment of the invention, the stress support system further comprises a plurality of markers attachable to the body part. The medical imaging device is configured to provide a dedicated sequence to track motion states and/or positions of the plurality of the attached markers. The medical imaging device is configured to perform a medical scan of the body part if desired motion states and/or desired positions of the plurality of the attached markers are reached.

For example, a fiducial marker may be used.

According to an embodiment of the invention, the dedicated sequence is at least one selected from: a navigator echo and a fast imaging sequence with a low resolution.

According to an embodiment of the invention, the image data of the series of medical scans are retrospectively reconstructed and sorted according to different motion states and/or different positions of the body part after image acquisition.

Alternative to the above-mentioned synchronization of scanning moment to dynamic motion, image series may be built up at both different levels of stress and at different positions of the body part. After image acquisition, the image data are sorted according to different motion states and/or different positions such that the image data are retrospectively reconstructed.

According to an embodiment of the invention, the medical imaging device is configured to initiate and/or terminate scanning based on a user input and/or a predetermined acute pain level.

In other words, the user, e.g. the patient or the healthcare professional, may control the scanning, e.g. start scanning when in pain and stop scanning when pain cannot be tolerated anymore. Alternatively or additionally, this may be done in an autonomous way by adding a pain sensing device to initiate and terminate scanning.

A third aspect of the invention relates to a method for operating a stress support system described above and below for medical imaging purposes. In a first step, a required stress level is determined for a medical scan. In a second step, a support device is attached to a patient for applying a physical stress to a body part of the patient. In a third step, a control device is used to modify the support device to adjust the applied physical stress to the required stress level.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 shows a schematic drawing of a stress support system according to an exemplary embodiment of the present disclosure.
Fig. 2 shows a schematic drawing of a medical imaging system according to an exemplary embodiment of the present disclosure
Fig. 3 shows a schematic drawing of a method for operating the stress support system according to an exemplary embodiment of the present disclosure

### DETAILED DESCRIPTION OF EMBODIMENTS

Fig. 1 schematically shows a stress support system 10 for diagnostic imaging according to an exemplary embodiment of the present disclosure. The stress support system 10 comprises a stress determination device 12 and at least one stress inducing device 14a, 14b. The at least one stress inducing device 14a, 14b comprises a support device 16a, 16b and a control device 18a, 18b.

The support device 16a, 16b is attachable to a patient and configured to apply a physical stress to a body part, such as a limb or the back, of the patient. In some implementations, the support device 16a, 16b may be a mechanical support device, such as a brace or an exoskeleton. In some implementations, the support device 16a, 16b may be an elasticated device, such as a tension strip or rubber band. In some implementations, the support device 16a, 16b may be a cycling-type device for inducing a dynamic physical stress. The support device 16a, 16b may be attached to the patient by a healthcare professional prior to the imaging.

The stress determination device 12 is configured to determine a required stress level for a medical scan of the body part. In some implementations, the stress determination device may determine the required stress level based on historical data of the patient or similar patients. For example, machine learning algorithm may be used to predict the required stress based on the historical data. In some implementations, the stress determination device may be coupled to a user interface via a physical cable or wirelessly to receive a user input to determine the required stress level.

The control device 18a, 18b is configured to modify the support device 16a, 16b to adjust the applied physical stress to the required stress level. The control device 18a, 18b may be a motorized device, which may be connected to the stress determination device 12 via a physical cable or wirelessly to receive instructions to adjust the applied physical stress. In case of a mechanical support device, e.g. a brace or exoskeleton, the control device 18a, 18b may adjust the position of e.g. a spring in order to increase or decrease the applied stress level. In case of an elasticated support device, e.g. a tension strip, rubber band, the control device 18a, 18b may adjust an anchoring position of a support of the elasticated support device for example by tightening or loosening a screw thread. In case of a cycling type device, the control device 18a, 18b may adjust a braking system to increase or decrease the load on the pedals.

In other words, the support device is attached to the patient prior to the imaging. During the imaging, the control device is configured to adapt the stress on the body part to the required stress level determined by the stress determination device. It is noted that the required stress level may be predetermined, i.e. determined prior to the imaging. Thus, during the imaging the stress induce device may adapt the static or dynamic stress in an autonomous manner with no or less operator interference. This may advantageously reduce the operator interference between scans, thereby increasing the patient throughput, i.e. number of patients that can be handled by a single diagnostic imaging apparatus per day, and preventing unwanted changes of the position of the patient.

The at least one stress inducing device 14a, 14b may optionally comprise a static stress inducing device 14a with a first support device 16a, e.g. a brace, a tension strip, and a first control device 18a. The first support device 16a is attachable to the patient and configured to apply a static physical stress to the body part. The stress determination device 12 is configured to determine a required static stress level for a medical scan. The first control device 18a is configured to modify the first support device 16a to adjust the applied static physical stress to the required static stress level.

The at least one stress inducing device 14a, 14b may optionally comprise a dynamic stress inducing device 14b with a second support device 16b, e.g. a brace, a tension strip, a cycling type device, and a second control device 18b. The second support device 16b is attachable to the patient and configured to apply a dynamic stress to the body part while being in motion. The stress determination device 12 is configured to determine a required dynamic stress level for a medical scan. The second control device 18b is configured to modify the second support device 16b to adjust the applied dynamic physical stress to the required dynamic stress level.

This may advantageously allow the body part being dynamically stressed, e.g. doing a cycling motion whilst being imaged in order to identify the problems with soft tissues, e.g. ligaments, muscles, cartilage. Additionally, the stress level is adapted for taking repeated images at different physical stress whilst in motion. As there is no or less operator interference between successive scans, the patient throughput is increased.

In addition to the stress value, the stress determination device 12 may optionally be configured to determine a required direction of the physical stress applied to the body part for a medical scan. The control device 18a, 18b is configured to modify the support device 16a, 16b to the required direction. For example, in case of a mechanical support device, this can be realized by pivoting a part of the mechanical support device to adjust the direction of applying the stress. In other words, also the direction of the stress inducing motion could be modified to scan, e.g. angular stress curve.

Optionally, the stress determination device 12 is configured to determine at least one of the following: a plurality of required stress levels for a series of medical scans and a minimum-maximum range of the required stress levels to be applied to the body part. The control device 18a, 18b is configured to modify the support device 16a, 16b to adjust the applied physical stress to the plurality of required stress levels for the series of medical scans and/or to adjust the applied physical stress within the minimum-maximum range of the required stress levels.

This may advantageously allow the level of stress to be adapted between successive scans of the body part, without any operator interference between scans. In case of a mechanical support device, the control device may be a motorized device for adjusting the position of e.g. a spring in order to increase the applied stress level between scans. In case of an elasticated support device, the control device may adjust an anchoring position of a support of the bend by tightening or loosening a screw thread.

At least one of the following may be determined based on historical data of the patient or similar patients: one or a plurality of the required stress levels for one or a series of medical scans, and a minimum-maximum range of the required stress levels to be applied to the body part. For example, machine learning algorithm may be used for define the minimum-maximum range based on the historical data. As a further example, machine learning algorithm may use the stress increase and decrease curves to predict the acceptable personalized stress levels for a series of medical scans. This may advantageously allow the stress determination device to determine these parameters automatically without any user input.

The stress support system 10 may optionally comprise a user interface 20. The user interface is configured to receive a user input to provide at least one of the following: defining at least one required stress level for a medical scan, defining a minimum-maximum range of the required stress levels to be applied to the body part, starting applying a physical stress, and stopping applying a physical stress. The stress determination device 12 is connectable to the user interface 20 via a physical cable or wirelessly and configured to determine one or a plurality of required stress levels and/or a minimum-maximum range of the required stress levels based on the user input. Alternatively or additionally, the control device 18a, 18b is connectable to the user interface 20 via a physical cable or wirelessly to start and stop modifying the support device 16a, 16b to apply the physical stress based on the user input.

The user interface 20 may be a patient interface. In order to avoid an excessive paint to the patient, whilst ensuring the corrected scans are obtained, the control of the level of stress may be given to the patient. For example, the patient may terminate a scan sequence if the increase of the stress causes excessive pain.

The user interface 20 may be a user interface for a healthcare professional for inputting one or multiple required stress levels and/or a minimum-maximum range of the required stress levels for one or a series of medical scans. The healthcare professional may also instruct the control device via the user interface to start or stop applying physical stress.

The stress support system 10 may optionally comprise at least one safety means 22. The at least one safety means 22 is configured to provide at least one safety parameter for limiting a maximum applied stress level. The control device 18a, 18b is configured to stop applying the physical stress to the body part of the patient or to re-adjust the applied physical stress level, if the at least one safety parameter matches a predefined criterion.

This may advantageously prevent the patient from experiencing an excessive pain and risk, particularly in the situations where the patient cannot easily express their pain to a healthcare profession - e.g. because they are unable to e.g. due to physical or mental limitations or there is no healthcare professional available. In other words, this may advantageously allow the stress support system to automatically take corrective action either to re-adjust the stress level or to stop applying physical stress to avoid pain once alerted by the safety means.

The at least one safety means 22 may optionally comprise an applied stress measuring device 22a configured to determine an absolute value of the applied stress as the at least one safety parameter. The control device 18a, 18b is configured to stop applying the physical stress to the body part of the patient or to re-adjust the applied physical stress level, if the absolute value matches a predefined criterion. The applied stress measuring device 22a may be a shear wave electrography or similar acousto-elasticity techniques that use an ultrasound sensor to measure the change in the stress of e.g. tissue, muscles, tendon before and during the imaging. The predefined criterion may be a predefined threshold. If the absolute value is larger than the predefined threshold, the control device 18a, 18b is configured to stop or re-adjust the applied physical stress.

The at least one safety means 22 may optionally comprise a pain sensing device 22b configured to measure a biometric signal indicative an acute pain level as the at least one safety parameter. The acute pain level is determined by the measured biometric signal based on a pain-biometrics model, which defines a relationship between the acute pain and a biometric signal based on historical data of the patient or similar patients. The control device 18a, 18b is configured to stop applying the physical stress to the body part of the patient or to re-adjust the applied physical stress level, if the determined acute pain level matches a predefined criterion.

The pain sensing device 22b may be a device which measures a vital sign which rapidly response to an acute pain. For example, GSC of the patient, which is measured using two electrodes and a high impedance amplifier attached to a part of the patient's body, may be used as the vital sign. The electrodes may be integrated into the fixation device - for example, the electrodes could be attached at a point on the inside of the fixation device where there is sufficient pressure applied to the patient's skin. Typically, the electrodes are of around 1cm² in area and separated by 1 to 5 cm. The pain sensing device 22b may be a device which measures another biometric signal indicative of the acute pain. For example, a camera device for measuring a facial expression or a cramping or spasm of the body may be used.

The pain sensing device 22b may be integrated in the support device. For example, the pain sensing device 22b may be located between the tissue of the patient and the support device, e.g. on the inside of the brace or exoskeleton. Alternatively, the pain sensing device may be worn separately at a preferred part of the patient's body - e.g. on the wrist, fingers, or foot, as here the number of sweat glands is higher.

The data from the pain sensing device 22b is passed through the pain-biometrics model to dynamically identify the acute pain level. The pain-biometric model may be based on machine learning techniques, such as regression and deep learning.

Optionally, the stress support system further comprises a residual stress sensing device 24. The residual stress sensing device is configured to measure a resultant stress due to an external pressure for correlating the determined acute pain level. The residual stress sensing device 24 may be an ultrasound sensor, e.g. attached to the limbs, to measure the resultant stress, and correlate to the level of pains using acousto-elasticity scans for re-construction of scan image. The ultrasound sensor may be a capacitive micromachined ultrasonic transducer (CMUT) which can be wrapped around limbs or muscle under stress.

The at least one safety means 22 may optionally comprise an image based feedback for providing at least one of the following as the at least one safety parameter: an imaging sequence, an image quality and an imaging parameter.

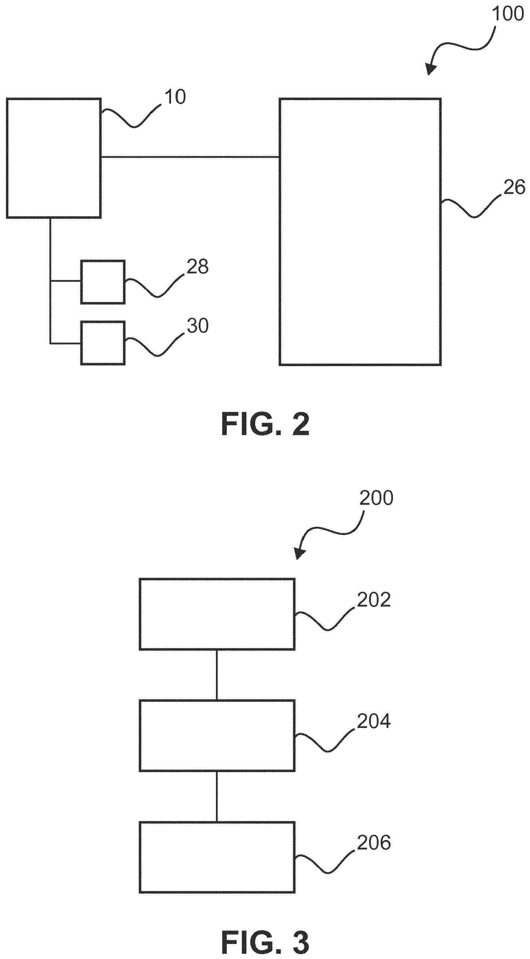

Manipulation may be realized within a certain parameter space, e.g. defined by the imaging sequence and image quality, and optionally by the pain level of the patient. Via an iterative feedback loop between the medical imaging device and the patient, increased image quality can be assured at a tolerable pain level of the patient. Imaging parameter, such as min/max distances of muscle, bone, etc. may also be used as limiting safety parameter. Fig. 2 schematically shows a medical imaging system 100 according to an exemplary embodiment of the present disclosure. The medical imaging system 100 comprises a stress support system as described above and below and a medical imaging device 26. The stress support system 10 is configured to apply a physical stress to a body part of a patient at a required stress level for a medical scan. The medical imaging device is configured to perform a medical scan of the body part to acquire image data, when the required stress level is reached.

In this way, the medical imaging device may perform a medical scan semiautomatically or automatically once the required stress level is reached. For example, the stress support system 10 may e.g. send a signal to the medical imaging device 26, once the required stress level is reached. This may e.g. trigger the medical imaging device to perform a medical scan.

The medical imaging device 26 may be a magnetic resonance imaging (MRI) scanner. The support device 16a, 16b as shown in Fig. 1 is MRI compatible. This may be achieved e.g. by using dedicated pressure sleeves that can force the limb into a predefined position when filled with air or liquid. In another example, the support device 16a, 16b may use non-magnetic material, e.g. polymers, for a mechanical brace or exoskeleton that can be operated by an extended axis from the control device 18a, 18b, which is outside the magnetic field. The medical imaging device 26 may be a computed tomography (CT) scanner. Non-metallic material e.g. carbon fiber based can be used for mechanical stability.

Optionally, the stress determination device 12 as shown in Fig. 1 is configured to determine a plurality of required stress levels for a series of medical scans. The medical imaging device 26 is configured to perform a series of medical scans of the body part at the plurality of required stress levels. The medical imaging device 26 is configured to be gated in a manner such that the image data of the series of medical scan are reconstructed at the same position of the body part.

This may be beneficial for imaging the body part under a dynamic physical stress, whilst the body part is both in motion and under stress. In this case, the image series need to be build up at both different levels of stress at the same position of the body part. Several approaches may be taken to achieve this:
In an example, the medical imaging device 26 is configured to perform a series of medical scans in synchronization with a motion state and/or a position of the body part. In other words, it is proposed to use dynamic stress, like cycling, and synchronize scanning moment to dynamic motion using extremely reproducible position. This can be achieved in various ways.

For example, a trigger signal may be provoked when the support device is in a predefined position. The medical imaging device 26 may be configured to perform a medical scan upon receiving the trigger signal.

As a further example, the medical imaging system 100 may comprise a motion tracking device 28. The motion tracking device 28 is configured to track a motion state and/or a position of the body part. The motion tracking device 28 may be a sensor or a marker that can be tracked by e.g. a camera.

As another example, the medical imaging system 100 may comprise a force sensing device 30. The force sensing device 30 is configured to measure a force between the body part and the attached support device 16a, 16b. The medical imaging device 26 is configured to perform a medical scan of the body part if a desired force is reached. The force sensing device 30 may be e.g. air pads. In other words, the image acquisition is activated when a certain force is created. The force sensing device 30 may be applied for static stress situation. For dynamic stress situation, the dynamic force is estimated based on the real-time conditions.

As a further example, the medical imaging device 26 is configured to provide a dedicated sequence to track a motion state and/or a position of the body part whilst being in motion. The medical imaging device 26 is configured to perform a medical scan of the body part if a desired motion state and/or a desired position is reached. The dedicated sequence may be at least one selected from a navigator echo or a fast imaging sequence with a low resolution. In other words, also the automatic image segmentation can trigger sequential scans in comparable positions. Also images from a former imaging session could be used to come to a comparable initial state for quantitative comparison of the patient's condition with the support of e.g. machine learning techniques applied to historical data. The machine learning techniques may include regression and neural network based deep learning with the historical data.

As a further example, the medical imaging device 26 comprises a plurality of markers (not shown) attachable to the body part. The medical imaging device 26 is configured to provide a dedicated sequence, e.g. navigator echo, to track motion states and/or positions of the plurality of the attached markers. The medical imaging device 26 is configured to perform a medical scan of the body part if desired motion states and/or desired positions of the plurality of the attached markers are reached.

Optionally, the image data of the series of medical scans are retrospectively reconstructed and sorted according to different motion states and/or positions of the body part after image acquisition. In other words, this may allow to capture image data built up at different levels of stress and at different positions of the body part. After image acquisition, the image data are sorted according to different motion states and/or positions to be retrospectively reconstructed.

The medical imaging device may be configured to initiate and/or terminate scanning based on a user input and/or a predetermined acute pain level. In other words, the user, e.g. the patient or the healthcare professional, may control the scanning, e.g. start scanning when in pain and stop scanning when the pain cannot be tolerated anymore. Alternatively or additionally, this may be done in an autonomous way by adding a pain sensing device to initiate and terminate scanning.

Fig. 3 schematically shows a method 200 for operating a stress support system according to an exemplary embodiment of the present disclosure. In step 202, a required stress level is determined for a medical scan. In step 204, a support device is attached to the patient for applying a physical stress to the body part. In step 206, the control device is used to modify the support device to adjust the applied physical stress to the required stress level.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A stress support system (10) for diagnostic imaging, comprising:
- a stress determination device (12); and
- at least one stress inducing device (14a, 14b) with a support device (16a, 16b) and a control device (18a, 18b);
wherein the support device is attachable to a patient and configured to apply a physical stress to a body part of the patient;
wherein the stress determination device is configured to determine a required stress level for a medical scan of the body part; and
wherein the control device is configured to modify the support device to adjust the applied physical stress to the required stress level.

2. Stress support system according to claim 1,
wherein the at least one stress inducing device comprises:
A) a static stress inducing device (14a) with a first support device (16a) and a first control device (18a);
wherein the first support device is attachable to the patient and configured to apply a static physical stress to the body part;
wherein the stress determination device is configured to determine a required static stress level for a medical scan; and
wherein the first control device is configured to modify the first support device to adjust the applied static physical stress to the required static stress level; and/or
B) a dynamic stress inducing device (14a) with a second support device (16a) and a second control device (18a);
wherein the second support device is attachable to the patient and configured to apply a dynamic stress to the body part while being in motion;
wherein the stress determination device is configured to determine a required dynamic stress level for a medical scan; and
wherein the second control device is configured to modify the second support device to adjust the applied dynamic physical stress to the required dynamic stress level.

3. Stress support system according to claim 1 or 2,
wherein the stress determination device is configured to determine a required direction of the physical stress applied to the body part for a medical scan; and
wherein the control device is configured to modify the support device to adjust the applied physical stress to the required direction.

4. Stress support system according to any of the preceding claims,
wherein the stress determination device is configured to determine at least one of the following:
- a plurality of required stress levels for a series of medical scans; and
- a minimum-maximum range of the required stress levels to be applied to the body part; and
wherein the control device is configured to modify the support device to adjust the applied physical stress to the plurality of required stress levels for the series of medical scans and/or to adjust the applied physical stress within the minimum-maximum range of the required stress levels.

5. Stress support system according to any of the preceding claims,
wherein at least one of the following is determined based on historical data of the patient or similar patients:
- one or a plurality of the required stress levels for one or a series of medical scans; and
- a minimum-maximum range of the required stress levels to be applied to the body part.

6. Stress support system according to any of the preceding claims, further comprising:
- a user interface (20);
wherein the user interface is configured to receive a user input to provide at least one of the following:
i) defining at least one required stress level for a medical scan;
ii) defining a minimum-maximum range of the required stress levels to be applied to the body part;
iii) starting applying a physical stress; and
iv) stopping applying a physical stress; and
wherein the stress determination device is connectable to the user interface and configured to determine one or a plurality of required stress levels and/or a minimum-maximum range of the required stress levels based on the user input; and/or
wherein the control device is connectable to the user interface and configured to start or stop modifying the support device to apply the physical stress based on the user input.

7. Stress support system according to any of the preceding claims, further comprising:
- at least one safety means (22);
wherein the at least one safety means is configured to provide at least one safety parameter for limiting a maximum applied stress level; and
wherein the control device is configured to stop applying the physical stress to the body part of the patient or to re-adjust the applied physical stress level, if the at least one provided safety parameter matches a predefined criterion.

8. Stress support system according to claim 7,
wherein the at least one safety means comprises an applied stress measuring device (22a) configured to determine an absolute value of the applied stress level as the at least one safety parameter; and
wherein the control device is configured to stop applying the physical stress to the body part of the patient or to re-adjust the applied physical stress level, if the absolute value of the applied stress level matches a predefined criterion.

9. Stress support system according to claim 7 or 8,
wherein the at least one safety means comprises a pain sensing device (22b) configured to measure a biometric signal indicative of an acute pain level of the patient as the at least one safety parameter;
wherein the acute pain level is determined by the measured biometric signal based on a pain-biometrics model, which defines a relationship between an acute pain and a biometric signal based on historical data of the patient or similar patients; and
wherein the control device is configured to stop applying the physical stress to the body part of the patient or to re-adjust the applied physical stress level, if the determined acute pain level matches a predefined criterion.

10. Stress support system according to claim 9, further comprising:
- a residual stress sensing device (24);
wherein the residual stress sensing device is configured to measure a resultant stress due to an external pressure for correlating the determined acute pain level.

11. Stress support system according to any of claims 7 to 10,
wherein the at least one safety means comprises an image based feedback for providing at least one of the following as the at least one safety parameter:
- an imaging sequence;
- an image quality; and
- an imaging parameter.

12. A medical imaging system (100), comprising:
- a stress support system according to any of claims 1 to 11; and
- a medical imaging device (26);
wherein the stress support system is configured to apply a physical stress to a body part of a patient at a required stress level for a medical scan; and
wherein the medical imaging device is configured to perform a medical scan of the body part to acquire image data, when the required stress level is reached.

13. Medical imaging system according to claim 12,
wherein the stress determination device is configured to determine a plurality of required stress levels for a series of medical scans; and
wherein the medical imaging device is configured to perform a series of medical scans of the body part at the plurality of required stress levels; and
wherein the medical imaging device is configured to be gated in a manner such that the image data of the series of medical scans are reconstructed at the same position of the body part.

14. Medical imaging system according to claims 13,
wherein the medical imaging device is configured to perform a series of medical scans in synchronization with a motion state and/or a position of the body part.

15. Medical imaging system according to claim 14,
wherein a trigger signal is provoked when the support device is in a predefined position; and
wherein the medical imaging device is configured to perform a medical scan upon receiving the trigger signal.

16. Medical imaging system according to claim 14 or 15, further comprising:
- a motion tracking device (28);
wherein the motion tracking device is configured to track a motion state and/or a position of the body part whilst being in motion; and
wherein the medical imaging device is configured to perform a medical scan of the body part if a desired motion state and/or a desired position is reached.

17. Medical imaging system according to any of claims 14 to 16, further comprising:
- a force sensing device (30);
wherein the force sensing device is configured to measure a force between the body object and the attached support device; and
wherein the medical imaging device is configured to perform a medical scan of the body part if a desired force is reached.

18. Medical imaging system according to any of claims 14 to 17,
wherein the medical imaging device is configured to provide a dedicated sequence to track a motion state and/or a position of the body part whilst being in motion;
wherein the medical imaging device is configured to perform a medical scan of the body part if a desired motion state and/or a desired position is reached;

19. Medical imaging system according to any of claims 14 to 18, further comprising:
- a plurality of markers attachable to the body part;
wherein the medical imaging device is configured to provide a dedicated sequence to track motion states and/or positions of the plurality of the attached markers; and
wherein the medical imaging device is configured to perform a medical scan of the body part if desired motion states and/or desired positions of the plurality of the attached markers are reached.

20. Medical imaging system according to claim 18 or 19,
wherein the dedicated sequence is at least one selected from:
- a navigator echo; and
- a fast imaging sequence with a low resolution.

21. Medical imaging system according to claim 13,
wherein the image data of the series of medical scans are retrospectively reconstructed and sorted according to different motion states and/or different positions of the body part after image acquisition.

22. Medical imaging system according to any of claims 12 to 21,
wherein the medical imaging device is configured to initiate and/or terminate scanning based on a user input and/or a predetermined acute pain level.

23. A method (200) for operating a stress support system according to any of claims 1 to 11 for diagnostic imaging, comprising the following steps:
- determining (202) a required stress level for a medical scan;
- attaching (204) a support device to a patient for applying a physical stress to a body part of the patient; and
- using (206) a control device to modify the support device to adjust the applied physical stress to the required stress level.
